(19) 

Europäisches Patentamt
European Patent Office
Office européen des brevets

(11) **EP 3 171 288 A1**

(12) **EUROPEAN PATENT APPLICATION**
published in accordance with Art. 153(4) EPC

(43) Date of publication:
**24.05.2017 Bulletin 2017/21**

(21) Application number: **15821867.7**

(22) Date of filing: **17.07.2015**

(51) Int Cl.:
*G06F 19/22* (2011.01)  *C12Q 1/68* (2006.01)

(86) International application number:
**PCT/KR2015/007461**

(87) International publication number:
**WO 2016/010401 (21.01.2016 Gazette 2016/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **18.07.2014  US 201462026283 P
31.12.2014  KR 20140194900**

(71) Applicant: **SK Telecom Co., Ltd
Seoul 100-999 (KR)**

(72) Inventor: **NAMKUNG, Junghyun
Seoul 100-999 (KR)**

(74) Representative: **Icely, Dominic Michael
The IP Asset Partnership Limited
Prama House
267 Banbury Road
Oxford OX2 7HT (GB)**

(54) **METHOD FOR PREDICTION OF FETAL MONOGENIC GENETIC VARIATIONS USING MATERNAL SERUM DNA**

(57) The present invention provides a method for non-invasively detecting, expecting, or diagnosing fetal single nucleotide polymorphisms and the resultant monogenic disorders, through maternal cell-free DNA sequencing. The diagnosis method according to the present invention does not harm mothers or fetuses and is convenient, in that analysis is possible using maternal blood samples; and can be favorably used for a prenatal diagnosis method capable of determining at an early stage whether single nucleotide polymorphisms causing monogenic disorders occur or not.

**FIG. 1**

Expected allele frequency in a duplication region

## Description

## Technical Field

The present disclosure relates to a method for predicting fetal monogenic genetic variation through the sequencing of maternal serum DNA. More particularly, the present disclosure relates to a method for the non-invasive prenatal prediction of fetal monogenic genetic variation and a resultant monogenic disorder from the sequencing of maternal serum DNA.

## Background Art

A recent increase in the incidence of congenital anomalies has focused attention on prenatal diagnosis, resulting in the development of various kinds of diagnostic equipment. Particularly, a pregnant women who 35 years old or older, who has delivered fetusren with chromosomal abnormalities, who was born with a structural chromosomal anomaly or whose fetus was conceived with a man with a structural chromosomal anomaly, who has a family history of genetic disorders, who is at risk of neural tube defect, or who is suspected of fetal deformity is recommended to receive prenatal diagnosis.

Prenatal diagnosis can be performed by invasive or non-invasive methods. Imposing an impact on the fetus, invasive diagnosis methods are in danger of causing miscarriage, disorders or deformity. Hence, non-invasive diagnosis methods have been developed as alternatives to overcome such problems.

The discovery of cell-free fetal DNA (cffDNA) in the cell-free DNA (cfDNA) of maternal plasma has provided a powerful tool for developing non-invasive prenatal genetic diagnosis methods. The application of cffDNA to prenatal diagnosis is further accelerated by the introduction of massively parallel sequencing, also called next-generation sequencing.

In addition, some studies showed that fetal and maternal DNAs are uniformly distributed throughout the entire genome, as analyzed by whole genome sequencing (WGS) and sequencing following the target enrichment of cffDNA (Lo YM et al., Science Translational Medicine 2010;2:61ra91; Liao GJ et al., Clinical Chemistry 2011;57:92-101; Kitzman JO et al., Science Translational Medicine 2012;4:137ra76). These results provide a basis on which detection can be extended to monogenic disorders, which are more common than chromosomal aneuploidy.

However, there are still many intricate obstacles to be overcome for application to monogenic disorders, whereas aneuploidy detection can be readily applied to clinical practice. Since the content of cffDNA in maternal plasma is low and very differs from one maternal plasma to another versatile, fetal variants are difficult to detect with reliability at the mononucleotide level.

## Disclosure

## Technical Problem

The present disclosure aims to provide a method for sensing, detecting, or diagnosing fetal monogenic variation through the sequencing of maternal serum DNA, or a method for predicting or diagnosing a monogenic disorder related to the monogenic variation or for providing information about the prediction/diagnosis of a monogenic disorder related to the monogenic variation.

## Technical Solution

The present disclosure addresses a method for predicting fetal monogenic genetic variation through the sequencing of maternal serum DNA. More particularly, the present disclosure addresses a method for the non-invasive prediction, detection or diagnosis of fetal monogenic genetic variation and a resultant monogenic disorder through the sequencing of maternal serum DNA.

An embodiment of the present disclosure provides a method for detecting a fetal genetic variation, using maternal serum RNA, the method comprising defining a genetic variation in a maternal single gene, with regard to a deletion- or duplication-type genetic mutation and a region where the genetic variation occurs, by use of sequencing analysis data of maternal serum DNA, and measuring an allele frequency of heterozygous SNP in the variation region from a maternal serum DNA having the genetic variation to determine whether or not the fetus has a deletion- or duplication-type genetic variation in the single gene linked to the monogenic disorder.

Another embodiment of the present disclosure addresses a method for detecting a fetal genetic variation, using maternal serum RNA, the method comprising defining a genetic variation in a maternal single gene, with regard to a deletion- or duplication-type genetic mutation and a region where the genetic variation occurs, by use of sequencing analysis data of maternal serum DNA, analyzing serum DNA and corpuscle DNA of the pregnant woman having the genetic variation through next-generation sequencing to acquire read depth ratios (serum read depth/corpuscle read depth) and applying the read depth ratios to determining whether or not the fetus has a deletion- or duplication-type genetic variation in the single gene linked to the monogenic disorder.

With the advance development of DNA sequencing techniques, massive genomic information can be deciphered. Particularly, next-generation sequencing (NGS)-based genomic analysis techniques are applied to the prenatal analysis. Based on the fact that maternal blood contains a fetal genome at a certain level, a maternal blood sample can be used to analyze fetal genomic information. This method is simple without impacts on the pregnant woman or the fetus, and can be useful as a prenatal diagnosis method by which a fetal genetic variation can be predicted in an early stage.

**Advantageous Effects**

Capable of predicting a fetal monogenic genetic variation before birth (from 6 weeks after conception) in a non-invasive manner through the sequencing of a serum DNA taken from a carrier pregnant woman, the method of the present disclosure is safe, effective and simple, and thus can be applied to prenatal diagnosis instead of conventional non-invasive methods, such as chorionic villus sampling and amniocentesis.

**Description of Drawings**

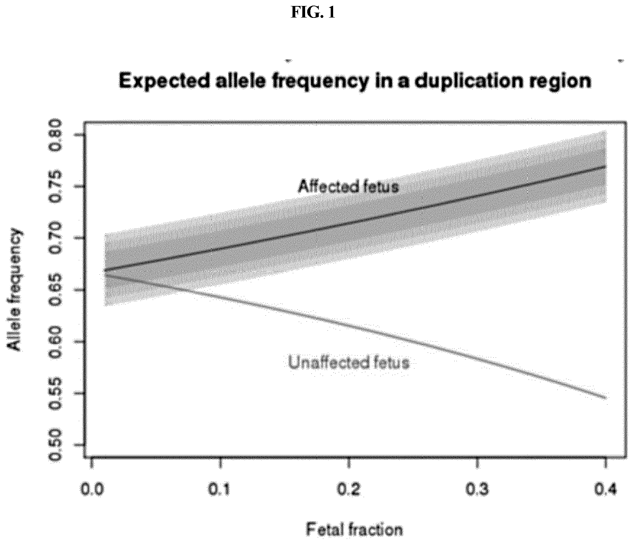

Fig. 1 shows plots of allele frequencies versus fetal DNA fractions, wherein the upper line accounts for a fetus having a mutation in a single gene and the lower line accounts for a fetus having no mutations in a single gene; and

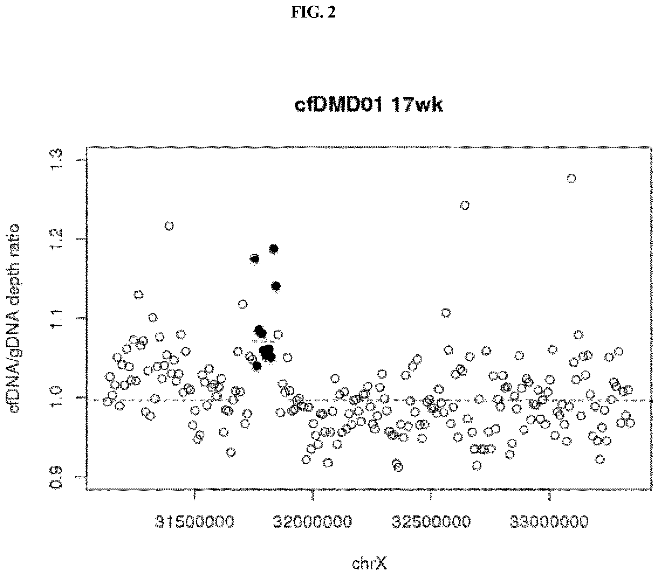

Fig. 2 shows ratios of serum read depth to corpuscle read depth (serum read depth/corpuscle read depth, $\gamma$)in variation and non-variation regions in a single gene, wherein the X-axis and Y-axis respectively represent nucleotide positions on an X chromosome and relative read depth ratios ($\gamma$) in variation regions (closed circles) and non-variation regions (open circles), together with a representative value in each region (dotted lines)

**Detailed Description**

The present invention will be described in more details.

The present disclosure addresses a method for sensing, detecting, or diagnosing fetal monogenic variation through the sequencing of maternal serum DNA. The method is simple because it does not need patrilineal or male-line DNA.

Particularly, the method for detecting fetal monogenic variation using maternal serum DNA comprises defining a genetic variation in a maternal single gene linked to a monogenic disorder, with regard to a deletion- or duplication-type genetic mutation and a region where the genetic variation occurs, by use of sequencing analysis data of maternal serum DNA (step A),

using a maternal serum DNA having a genetic variation to analyze a discriminate genetic trait depending on fetal genetic variation in the variation region or non-variation region (normal region)(step B), and

determining whether or not the fetus has a deletion- or duplication-type genetic variation in the single gene linked to the monogenic disorder (step C).

In accordance with some embodiments of the present disclosure, the step B of using a maternal serum DNA having a genetic variation to analyze a discriminate genetic trait can be carried out in two manners. First, the maternal serum DNA having a genetic variation is measured for the allele frequency of heterozygous SNP in the variation region to give a measurement distribution of the allele frequency. A fetal DNA fraction (*f*) of the maternal serum DNA is obtained, and used, together with Mendel's Law, to calculate expectation values of the allele frequency of heterozygous SNP in the variation region of the single gene for the presence and absence of fetal genetic variation. Then, the measurement distribution and the expectation values are used to determine whether or not the fetus has a genetic mutation.

Second, maternal serum DNA and corpuscle DNA (genomic DNA) is analyzed through next-generation sequencing to acquire read depths in a variation region and a non-variation region of each of the serum DNA and the corpuscle DNA, and ratios of serum read depth to corpuscle read depth (serum read depth/corpuscle read depth) are compared to determine the presence or absence of a fetal genetic mutation.

In accordance with some embodiments of the present disclosure, the method of using an allele frequency of heterozygous SNP in a genetic variation region may be carried out as follows:

analyzing the maternal serum DNA to give maternal sequencing data;

defining a deletion- or duplication-type genetic mutation and a variation region in a single gene linked to a maternal monogenic disorder from the sequencing data;
measuring an allele frequency of heterozygous SNP in the variation region from a maternal serum DNA having the genetic variation to give a measurement distribution of the allele frequency;
obtaining a fetal DNA fraction ($f$) of the maternal serum DNA, and applying Mendel's Law to calculate an expectation value of the allele frequency of heterozygous SNP in the variation region of the single gene as a first expectation value ($\theta_{exp|aff}$) when the fetus has the genetic variation of the single gene and as a second expectation value ($\theta_{exp|unaff}$) when the fetus does not have the genetic variation of the single gene; and
determining whether the fetus has a monogenic deletion or duplication mutation for a monogenic disorder, based on whether the first expectation value ($\theta_{exp|aff}$) and the second expectation value ($\theta_{exp|unaff}$) fall within a statistically significant interval of the measurement distribution of the allele frequency.

The detecting method of the present disclosure will be stepwise explained in more detail.

**(Step A)**

This step is adapted to define a genetic variation in a maternal single gene linked to a monogenic disorder, with regard to a deletion- or duplication-type genetic mutation and a region where the genetic variation occurs, by use of sequencing analysis data of maternal serum DNA

Since the method of the present disclosure is based on the analysis of an allele frequency for an X-linked disorder, that is, a monogenic disorder, from a maternal serum, it will be readily understood to those skilled in the art that the method of the present disclosure can be applied to an X-linked recessive disorder or an autosomal recessive disorder.

Examples of the disorders caused by monogenic genetic variations may be monogenic disorders including, but not limited to, Duchenne muscular dystrophy, Pelizaeus-Merzbacher disease, myotubular myopathy, Lowe syndrome, Menkes syndrome, X-linked adrenoleukodystrophy, Hoyeraal-Hreidarsson syndrome, spinal muscular atrophy, metachromatic leukodystrophy, and Krabbe disease.

As used herein, the term "carrier pregnant woman" or "target pregnant woman" refers to a pregnant woman having a single-gene mutation in an X chromosome. Because the carrier pregnant woman has a recessive single-gene mutation (X') in only one of her two X (XX) chromosomes, she does not express the monogenic disorder. In the present disclosure, the carrier pregnant woman is particularly one at 6 weeks or longer after conception. The cell-free DNA of the carrier pregnant woman includes cell-free fetal DNA.

Whether or not a pregnant woman is a carrier can be determined by investigating the family story of a corresponding single-gene disease or sequencing genomic DNA and comparing the sequence with a reference DNA sequence, which is a normal DNA sequence or has a single-gene mutation.

A pregnant woman who is a carrier and from whom cell-free DNA can be obtained during a pregnancy period is an experimental target in the present discbsure

The sequencing of serum DNA may be performed by a next-generation sequencing method, examples of which include, but are not limited to, target enrichment and massively parallel sequencing.

Types (insertion or deletion) and regions of genetic mutations can be revealed using many well-known structural variation-detecting programs. Examples of such structural variation-detecting programs include Delly, Pindell, Break-Dancer, GASV, Hydra, and CNVnator, but are not limited thereto. In a particular embodiment, the type and region of a genetic mutation may be defined by calculating a moving average from a read depth for an overlapping sliding window having a window size of 10 kb read points after massively parallel sequencing; and applying a CBS (circular binary segmentation) algorithm. For example, the definition of a deletion- or duplication-type genetic mutation and a variation region in a single gene can be performed by calculating a moving average from a read depth obtained from the analysis of maternal serum DNA through next-generation sequencing, and applying CBS (circular binary segmentation). Optionally, the method may further comprise a step of eliminating variation attributed to GC content differences according to gene regions from the read depth data, using a known method, in advance.

**(Steps B and C)**

Step B utilizes the allele frequency of heterozygous SNP in the genetic variation region of maternal serum DNA, and is largely divided into the utilization of SNP allele frequency (B-1) and the utilization of a ratio of serum read depth to corpuscle read depth in variation and non-variation regions of a single gene (serum read depth/corpuscle read depth) (B-2).

According to a particular embodiment, the alleles used to obtain a measurement distribution and an expectation value of the allele frequency are the same. For instance, a minor allele with an allele frequency less than 0.5 or a major allele with an allele frequency greater than 0.5 may be used in both steps of obtaining a measurement distribution of the allele frequency and an expectation value of the allele frequency. When the minor allele and the major allele are used together to obtain a measurement distribution and an expectation value of the allele frequency, both the measurement value and the expectation value converged at 0.5, regardless of fetal genetic variation, thus making not only the difference based on the assumption small but also complicating the calculation of the expectation value.

A first substep (B-1) of the step B, which is adapted to utilize an SNP allele frequency, may be carried out as follows.

(B-11) measuring maternal serum DNA having a genetic variation for an allele frequency of heterozygous single nucleotide polymorphism (SNP) in the variation region to give a measurement distribution of the allele frequency,

(B-12) obtaining a fetal DNA fraction ($f$) of the maternal serum DNA, and applying Mendel's Law to calculating an expectation value of the allele frequency of heterozygous SNP in the variation region of the single gene as a first expectation value ($\theta_{exp|aff}$) when the fetus has a genetic variation in the single gene and as a second expectation value ($\theta_{exp|unaff}$) when the fetus does not have a genetic variation in the single gene, and

(B-13) determining whether the fetus has a monogenic deletion or duplication mutation for a monogenic disorder on the basis of the fact that the first expectation value ($\theta_{exp|aff}$) and the second expectation value ($\theta_{exp|unaff}$) fall within a statistically significant interval of the measurement distribution of the allele frequency.

Optionally, substep B-11 may further comprise eliminating outliers from the allele frequency values using a known process.

Substep B-11 is designed to measure an average allele frequency ($\theta_{obs}$) of heterozygous SNP in a DNA region having a monogenic genetic variation (duplication/deletion) from maternal serum DNA sequence data. The allele frequency can be calculated by counting the number of each allele in next-generation sequencing data by use of a conventional program such as Samtools.

In substep B-12, a fetal DNA fraction ($f$) in the serum DNA of a carrier pregnant woman is obtained. The fetal DNA fraction may be measured using one of various conventional methods.

By way of example, a male fetal DNA fraction (f) of maternal serum DNA may be obtained by capturing ZFX and ZFY genes with capture probes that can respectively capture an X-linked zinc finger (Zfx) gene region and a Y-linked zinc finger (Zfy) gene region. For a male fetus, in greater detail, custom capture probes that target ZFX (X-linked zinc finger protein) and ZFY (Y-linked zinc finger protein) gene regions may be used to capture ZFX and ZFY genes, followed by calculation according to the following equation:

$$\text{Fetal DNA fraction: } f = \frac{2ZFY'}{ZFX' + ZFY'}$$

wherein, ZFX' and ZFY' are the quotients of the number of total mapped reads divided by the number of probes.

A female fetal DNA fraction (f) of the maternal serum DNA can be obtained using a distribution of a SNP allele frequency, formed over an allele frequency range from 0.02 to 0.3, with 0.1 as a median in a non-variation region.

In substep B-12, a fetal DNA fraction ($f$) of the maternal serum DNA is obtained, and an expectation value of the allele frequency of heterozygous SNP in the variation region of the single gene is calculated as a first expectation value ($\theta_{exp|aff}$) when the fetus has a genetic variation in the single gene and as a second expectation value ($\theta_{exp|unaff}$) when the fetus does not have a genetic variation in the single gene, using Mendel's Law. The equations according to which the first expectation value and the second expectation values are calculated can be established depending on the kind of chromosome (e.g., autosomes and allosomes), the kind of Mendelian inheritance, and the sex of the fetus in the case of allosomes, under the assumption that the pregnant woman is the carrier.

For example, when the fetus is male with an X-linked recessive disorder, the first expectation value ($\theta_{exp|aff}$) and the second expectation value ($\theta_{exp|unaff}$) can be calculated according to the following Equations 1 and 2:

<Equation 1>

$$\theta_{exp|aff} = 2/(3-f)$$

<Equation 2>

$$\theta_{exp|unaff} = 2(1-f)/3-2f$$

Substep B-13 is designed to determine whether the fetus has a monogenic deletion or duplication mutation for a monogenic disorder based on the fact that the first expectation value ($\theta_{exp|aff}$) and the second expectation value

($\theta_{exp|unaff}$) fall within a statistically significant interval of the measurement distribution of the allele frequency.

For instance, when the measured average allele frequency ($\theta_{obs}$) is nearer to the expectation value ($\theta_{exp|aff}$) of the allele frequency for a fetus that has a monogenic genetic variation, the fetus in the carrier pregnant woman is predicted to have a monogenic genetic variation. On the other hand, when the measured average allele frequency ($\theta_{obs}$) is nearer to the expectation value ($\theta_{exp|unaff}$) of the allele frequency for a fetus that does not have a monogenic genetic variation, the fetus in the carrier pregnant woman is predicted not to have the monogenic genetic variation. For statistical significance, the region of significance of the measured allele frequency ($\theta_{obs}$) is calculated under the assumption that it is binomially distributed. If both the expectation values are within the region of significance, the prediction is determined to be without statistical significance.

In accordance with another embodiments of the present disclosure, the second substep B-2 of the step B, which is designed to use the ratio of serum read depth to corpuscle read depth in variation and non-variation regions of a single gene (serum read depth/corpuscle read depth), may be carried out by:

defining a genetic variation in a single gene for a maternal monogenic disorder, with regard to the kind of genetic mutation (deletion or duplication) and the region where the genetic variation occurs, by use of the sequencing analysis data of maternal serum DNA;
acquiring, from the serum DNA sequencing data of the pregnant woman having the genetic variation, respective serum read depths in a variation region and a non-variation region in a single gene;
acquiring, from the corpuscle DNA sequencing data of the pregnant woman having the genetic variation, respective corpuscle read depths in a variation region and a non-variation region in the single gene;
calculating a first ratio of the serum read depth to the corpuscle read depth (serum read depth/corpuscle read depth) in the variation region in the single gene and a second ratio of the serum read depth to the corpuscle read depth (serum read depth/corpuscle read depth) in the non-variation region in the single gene; and
comparing the first ratio with the second ratio to determine whether or not the fetus has a deletion- or duplication-type genetic mutation, which may cause a monogenic disorder, in the single gene.

Optionally, the substeps of acquiring the first and second ratios may further comprise eliminating variation attributed to GC content differences according to gene regions from the read depth data, using a known method, and eliminating an outlier of the allele frequency values from the read depth data, using a known method.

In the step of defining genetic variation with regard to kind and region by use of the sequencing analysis data of maternal serum DNA, the sequence information of maternal serum DNA is analyzed and used to define the kind of genetic variation (deletion or duplication) and the region of the genetic variation in the single gene, which may account for the monogenic disorder of the pregnant woman. The sequence analysis of maternal serum DNA may be carried out by obtaining maternal serum DNA and sequencing the serum DNA. The sequence analysis of serum DNA and the determination of the kind and region of genetic variation may be carried out in the same manner as in step A.

The serum DNA and genome DNA of a carrier pregnant woman having a monogenic genetic variation may be analyzed using next-generation sequencing (NGS) to determine the kind and region of monogenic genetic variation, as described above. This method may be applied to both duplication and deletion mutations. All ofthe disorder, the pregnant woman, and the single gene that are used in step B-2 can be treated in the same manner as in step A.

In detail, the utilization of a ratio of serum read depth to corpuscle read depth in variation and non-variation regions of a single gene (serum read depth/corpuscle read depth) may be implemented by:

(B-21) acquiring, from the serum DNA sequencing data ofthe pregnant woman having the genetic variation, respective serum read depths in a variation region and a non-variation region in the single gene;
(B-22) acquiring, from the corpuscle DNA sequencing data of the pregnant woman having the genetic variation, respective corpuscle read depths in a variation region and a non-variation region in the single gene;
(B-23) calculating a first ratio of the serum read depth to the corpuscle read depth (serum read depth/corpuscle read depth) in the variation region in the single gene and a second ratio of the serum read depth to the corpuscle read depth (serum read depth/corpuscle read depth) in the non-variation region in the single gene; and
(B-24) comparing the first ratio with the second ratio to determine whether or not the fetus has a deletion- or duplication-type genetic mutation, which may cause a monogenic disorder, in the single gene.

For use in substep B-21 of acquiring, from the serum DNA sequencing data of the pregnant woman having the genetic variation, respective serum read depths in a variation region and a non-variation region in the single gene, the sequence information and the read depths may be obtained using next-generation sequencing, as described in step A. For example, a next-generation sequencing method including target enrichment and massively parallel sequencing may be used.

Substep B-22 of acquiring, from the corpuscle DNA sequencing data of the pregnant woman having the genetic variation, respective corpuscle read depths in a variation region and a non-variation region in the single gene maybe carried out in the same manner as in substep B-21, with the exception of using corpuscle DNA instead of serum DNA. For example, maternal corpuscle DNA is obtained and then analyzed for sequence and read depth using next-generation sequencing.

Maternal serum and corpuscle DNA may be obtained by separating a maternal blood sample into a serum and corpuscles and isolating respective DNA from the serum and the corpuscles. The corpuscles can be obtained by, without limitation thereto, centrifuging a blood sample from the pregnant women, and the corpuscles may be lysed to extract genome DNA. DNA isolation may be performed using a conventional method well known in the art. Separation of maternal blood samples into serum and corpuscles makes it possible to obtain both serum and corpuscles from a blood sample taken only once from a pregnant woman, so that the method is simple. In addition, the method of the present disclosure enjoys the advantage of requiring no patrilineal DNA, and detecting fetal genetic variation with high sensitivity and accuracy.

According to a particular embodiment, the maternal serum and corpuscle DNA may be analyzed using next-generation sequencing with the same custom capture probes under the same conditions in order to minimize measurement errors. When target single genes are determined, the custom capture probes can be easily selected, and thus are not particularly limited.

In substeps B-23 and B-24, the first ratio of serum read depth to corpuscle read depth in a variation region of the single gene (serum read depth/corpuscle read depth) and the second ratio of serum read depth to corpuscle read depth in a non-variation region of the single gene (serum read depth/corpuscle read depth) are calculated and compared in order to determine whether or not the fetus has a deletion- or duplication-type genetic mutation, which is likely to cause a monogenic disorder, in the single gene.

In detail, a relative serum read depth is calculated by dividing a read depth for maternal serum DNA by a read depth for maternal genome DNA in a variation region. Likewise, a relative serum read depth is calculated by dividing a read depth for maternal serum DNA by a read depth for maternal genome DNA in a non-variation region. Read depths may be calculated using conventional programs, such as Samtools, and relative read depths are calculated according to base sequence positions.

According to some embodiments of the present disclosure, the first ratio may be a mean or median value of two or more measurements obtained from individual nucleotides in the variation region in the single gene, while the second ratio may be a mean or median value of two or more measurements obtained from individual nucleotides in the non-variation region in the single gene.

Alternatively, the first ratio may be a mean or median value of two or more measurements of the moving average of read depth obtained from individual nucleotide bins of 5 to 100,000 bases in the variation region in the single gene while the second ratio may be a mean or median value oftwo or more measurements ofthe moving average of read depth obtained from individual nucleotide bins of 5 to 100,000 bases in the non-variation region in the single gene. For computation of the moving average of read depths, the nucleotide bins may or may not be set to overlap.

The first ratio is compared with the second ratio to determine whether or not the fetus has a deletion- or duplication-type genetic mutation, which may cause a monogenic disorder, in the single gene. In this regard, when the maternal genetic variation is of a deletion type and the first ratio for the variation region is smaller than the second ratio for the non-variation region, the fetus can be determined to have a deletion variation in the single gene. On the other hand, when the maternal genetic variation is of a duplication type and the first ratio for the variation region is larger than the second ratio for the non-variation region, the fetus can be determined to have a duplication variation in the single gene.

For example, when a woman with a monogenic genetic variation conceives a male fetus, the ratio of serum DNA read depth to genome DNA read depth (relative read depth: $\gamma$) varies depending on whether or not the fetus has a monogenic genetic variation and on the kind of fetal monogenic genetic variation, if present (see Table 5).

As can be seen in Table 5, when the fetus has a deletion mutation, $\gamma$ in the variation region is smaller than that in a normal region. When the fetus has no deletion mutations, $\gamma$ in the variation region is larger than that in a normal region. For a fetal duplication mutation, $\gamma$ in the variation region is larger than that in a normal region. On the other hand, when the fetus has no duplication mutations, $\gamma$ in the variation region is smaller than that in a normal region. With respect to a pregnant woman having a deletion mutation, the fetus is determined to have a deletion genetic mutation when the first ratio for the variation region is smaller than the second ratio for the non-variation region. The fetus in a pregnant woman having a duplication mutation can be determined to have a duplication mutation in a single gene when the first ratio for the variation region is larger than the second ratio for the non-variation region.

In greater detail, when the first ratio is compared to the second ratio for a pregnant woman having a deletion mutation, a comparison result in which a relative read depth in a variation region in the single gene is smaller than that in a non-variation region in the single gene makes it possible to predict that the fetus might have a monogenic genetic variation. On the other hand, a comparison result in which a relative read depth in a variation region in the single gene is larger than that in a non-variation region in the single gene leads to the prediction that the fetus might have no monogenic genetic variations.

For a pregnant woman having a duplication mutation, when a relative read depth in a variation region in the single gene is found to be larger than that in a non-variation region in the single gene as the result of comparison between the first ratio and the second ratio, the fetus is predicted to have a monogenic genetic variation. In contrast, the fetus is predicted to have no monogenic genetic variations when a relative read depth in a variation region in the single gene is smaller than that in a non-variation region in the single gene.

As described above, the method of the present disclosure is carried out by determining whether or not a pregnant woman has a deletion or duplication mutation in a single gene, followed by comparing $\gamma$ in a normal region with $\gamma$ in a variation region, whereby the inheritance by the fetus of the single-gene mutation, that is, whether the fetus has the single-gene mutation, can be easily determined.

As such, the method according to some embodiments of the present disclosure is designed to precisely predict the single-gene mutation of a fetus by analyzing maternal serum and genome DNA using next-generation sequencing.

Hence, the present disclosure can provide diagnostic information about fetal monogenic disorders using maternal serum DNA in determining a deletion or duplication mutation in a single gene.

A better understanding of the present invention may be obtained through the following examples which are set forth to illustrate, but are not to be construed as limiting the present invention.

### EXAMPLE 1: Prediction of Fetal Monogenic Variation

#### <1-1> Sequencing of maternal serum DNA

As the monogenic disorder that was handled in this experiment, an X-linked recessive disorder was selected.

Maternal serum DNA was assumed to have a duplication mutation, causative of a genetic disorder, in some region.

It was assumed that a pregnant woman showed a target sequencing coverage of about 95 %, an average serum DNA read depth of about 100, and an average genome DNA read depth of about 100.

#### <1-2> Identification of kind and region of genetic variation

To identify the kind and region of a single-gene mutation, the moving average of read depths was computed and processed using a CBS algorithm. As a result, the region was determined and the pregnant woman was found to have a duplication mutation.

#### <1-3> Measurement of fetal DNA fraction

The fetal DNA content of maternal DNA was measured as in Example <1-1>. To this end, ZFX and ZFY genes were captured using an Agilent SureSelect Custom Kit containing capture probes that target ZFX (X-linked zinc finger protein) and ZFY (Y-linked zinc finger protein) regions.

$$f = \frac{2ZFY\prime}{ZFX\prime + ZFY\prime}$$

Fetal DNA fraction:

wherein, ZFX' and ZFY' are the quotients of the number of total mapped reads divided by the number of probes.

Results of prediction of fetal DNA fraction (*f*) using ZFX and ZFY genes are given in Table 1, below.

TABLE 1

| Sample | number of total mapped reads | | Predicted fetal DNA fraction |
|---|---|---|---|
| | ZFX | ZFY | |
| Mother I-2 | 24242485 | 878522 | 0.08604 |
| Mother I-1 | 44040616 | 1175572 | 0.06484 |

As can be seen in Table 1, the fetal DNA fraction was 8.6 % in the serum of pregnant woman I-1 and 6.4 % in the serum of pregnant woman II-2.

**<1-4> Prediction of allele frequency of heterozygous single-nucleotide polymorphism**

Assuming that each of the pregnant women had a duplication mutation carrier and conceived a male fetus, the fetal DNA fraction $f$ may be used to derive an allele frequency ($\theta_{exp/aff}$) of heterozygous SNP in a variation region in a single gene according to the following Equation 1 and an allele frequency ($\theta_{exp/unaff}$) of heterozygous SNP in a non-variation region in the single gene according to the following Equation 2:

<Equation 1>

$$\theta_{exp/aff} = 2/(3-f)$$

<Equation 2>

$$\theta_{exp/unaff} = 2(1-f)/(3-2f)$$

In Fig. 1, allele frequencies are plotted versus fetal DNA fractions. The upper line indicates a fetus having a single-gene mutation while the lower line accounts for a fetus having no single-gene mutations. The gray regions along the upper line show a confidence interval calculated from an allele frequency of 12 SNPs in the variation region and standard error resulting from 100 rounds of calculation of random samples of consecutive 6 and 3 SNPs.

When the fetal DNA fractions calculated in Example 1-3 were input into the equations, allele frequencies were predicted as given in Table 2, below.

Table 2

| Item | Mother I-1 | Mother I-2 |
|---|---|---|
| fetal DNA fraction ($f$, %) | 8.604% | 6.484% |
| ($\theta_{exp/aff}$) (fetus with genetic mutation) | 0.6863512 | 0.6813939 |
| ($\theta_{exp/unaff}$) (fetus without genetic mutation) | 0.6463832 | 0.6516068 |

**<1-5> Measurement of average allele frequency ($\theta_{obs}$) of heterozygous SNP in DNA region having single-gene mutation**

From next-generation sequencing data of maternal serum DNA, the average allele frequency ($\theta_{obs}$) of heterozygous SNP in a DNA region having a single-gene mutation can be calculated by counting the number of each allele gene. The calculation results from the data of Examples are given in Table 3, below.

TABLE 3

| | Mother I-1 | Mother I-2 |
|---|---|---|
| Number of heterozygous SNP | 12 | 10 |
| $\theta_{obs}$ (observed average allele frequency) (95% confidence interval) | 0.6782778 (0.6606647-0.6958908) | 0.6826352 (0.69998752-0.6653952) |

Because the calculated average allele frequency ($\theta_{obs}$) of SNP in Table 3 was nearer to the calculated ($\theta_{exp/aff}$) values for mutation regions in Table 2, the fetus conceived by the pregnant woman can be predicted to have a single-gene mutation.

**EXAMPLE 2: Prediction of Fetal Monogenic Variation**

**<2-1> Sequencing of maternal serum DNA**

It was assumed that a pregnant women had a deletion mutation causative of a recessive genetic disorder and conceived a male fetus.

Serum and genome DNAs from the pregnant woman were analyzed using massively parallel sequencing, a kind of next-generation sequencing, in the same manner as in Example 1-1.

The pregnant woman showed a target sequencing coverage of about 97.7 %, an average serum DNA read depth of from about 465 to about 530, and an average genome DNA read depth of about 1210.

<2-2> Identification of kind and region of genetic variation

To identify the kind and region of a single-gene mutation, the moving average of read depths was computed and subjected to CBS algorithm.

<2-3> Measurement of fetal DNA fraction

The fetal DNA content of maternal DNA was measured as in Example <1-3>. The measurement results are shown in Table 4, below.

TABLE 4

| Sample | number of total mapped reads | | Predicted fetal DNA fraction |
|---|---|---|---|
| | ZFX | ZFY | |
| Mother II-1 | 26051623 | 567051 | 0.05352 |
| Mother II-2 | 29480725 | 888942 | 0.07264 |

As can be seen in Table 4, the fetal DNA fraction was 5.4 % in the serum of pregnant woman II-1 and 7.3 % in the serum of pregnant woman II-2.

<2-4> Ratio of read depth between serum DNA and corpuscle DNA (genome DNA) in normal (non-variation) and variation regions

Assuming that each of the pregnant women had a single-gene mutation carrier and was carrying a male fetus, the ratio of serum DNA read depth to genome DNA read depth may be predicted, as shown in Table 5, depending on whether the fetus has a single-gene mutation.

TABLE 5

| Mutation status | normal | deletion | | duplication | |
|---|---|---|---|---|---|
| Read depth of mother gDNA | 1 | 0.5 | | 1.5 | |
| cfDNA | | Fetus status | | Fetus status 태 | |
| | | With mutation | Without mutation | With mutation | Without mutation |
| Read depth of mother DNA | 1-$f$ | (1-$f$)/2 | (1-$f$)/2 | 3(1-$f$)/2 | 3(1-$f$)/2 |
| Read depth of fetus DNA | $f$/2 | 0 | $f$/2 | $f$ | $f$ |
| Read depth of cfDNA | (2-$f$)/2 | (1-$f$)/2 | 1/2 | (3-$f$)/2 | (3-2$f$)/2 |
| $\gamma$(cfDNA/gDNA) | (2-$f$)/2 =1-($f$/2) | 1-$f$ | 1 | (3-$f$)/3 =1-($f$/3) | (3-2$f$)/3 =1-(2$f$/3) |
| Comparison of read ratio | | Lower than normal | Higher than normal | Higher than normal | Lower than normal |
| $f$: fetal DNA fraction | | | | | |

As can be seen in Table 5, when the fetus has a deletion mutation, $\gamma$ in a variation region is smaller than that in a normal region. On the other hand, when the fetus has no deletion mutations, $\gamma$ in a variation region is larger than that in a normal region. Hence, when the maternal single-gene mutation is of a deletion type, comparison of $\gamma$ between a normal region and a variation region allows for determining the inheritance of the single-gene mutation from the pregnant woman to the fetus, that is, whether or not the fetus has the single-gene mutation.

In addition, when the fetus has a duplication mutation, $\gamma$ in a variation region is larger than that in a normal region. On the other hand, in the absence of fetal deletion mutations, $\gamma$ in a variation region is smaller than that in a normal region. Hence, when the maternal single-gene mutation is of a duplication type, comparison of $\gamma$ between a normal region and a variation region allows for determining the inheritance of the single-gene mutation from the pregnant woman to the fetus, that is, whether or not the fetus has the single-gene mutation.

Based on the prediction, ratios of serum DNA read depth to corpuscle DNA read depth were measured in normal and variation regions.

As an experimental result, the relative read depths ($\gamma$) were 0.99632 and 1.06031 in a normal region and a variation region of pregnant woman II-1, respectively. For pregnant woman II-2, the relative read depths ($\gamma$) were 0.99647 in a normal region and 1.07075 in a variation region. Relative read depths ($\gamma$) for pregnant woman II-2 are depicted in Fig. 2. In Fig. 2, the X-axis represents nucleotide positions on an X-chromosome while the Y-axis accounts for relative read depths ($\gamma$). Read depth ratios in variation regions (closed circles) and non-variation regions (open circles) are given, together with a representative value (dotted lines) in each region.

For pregnant woman II-1 having a deletion mutation, the fetus was predicted to be normal as the relative read depths in variation regions were larger than those in normal regions.

**Claims**

1. A method for detecting fetal genetic variation using a maternal serum DNA, comprising:

   analyzing a maternal serum DNA to give a maternal sequencing data;
   defining a genetic mutation type of deletion or duplication and a variation region in a single gene linked to a maternal monogenic disorder using the sequencing data;
   obtaining a distribution ofthe measured allele frequency by measuring an allele frequency of heterozygous SNP in the variation region of a maternal serum DNA having the genetic variation;
   obtaining an expectation value of the allele frequency of heterozygous SNP in the variation region ofthe single gene as a first expectation value ($\theta_{exp|aff}$) when the fetus has the genetic variation of the single gene and as a second expectation value ($\theta_{exp|unaff}$) when the fetus does not have the genetic variation of the single gene, by using a fetal DNA fraction ($f$) of the maternal serum DNA, and applying Mendel's Law; and
   determining whether the fetus has a genetic mutation of deletion or duplication in the single gene linked to a monogenic disorder, based on whether the first expectation value ($\theta_{exp|aff}$) and the second expectation value ($\theta_{exp|unaff}$) fall within a statistically significant interval of the distribution ofthe measured allele frequency.

2. The method of claim 1, wherein the step of analyzing the maternal serum DNA is performed using a next-generation sequencing method including target enrichment and massively parallel sequencing.

3. The method of claim 1, wherein the step of defining a genetic mutation type of deletion or duplication and a variation region in a maternal single gene is performed by calculating a moving average from a read depth obtained from analysis of maternal serum DNA through next-generation sequencing and by applying CBS (circular binary segmentation).

4. The method of claim 1, wherein the allele used in the steps of obtaining a distribution of the measured allele frequency and obtaining an expectation value of the allele frequency is a minor allele with an allele frequency less than 0.5.

5. The method of claim 1, wherein the allele used in the steps of obtaining a distribution of the measured allele frequency and obtaining an expectation value of the allele frequency is a major allele with an allele frequency greater than 0.5.

6. The method of claim 1, wherein a male fetal DNA fraction ($f$) ofthe maternal serum DNA is obtained by capturing ZFX and ZFY genes with respective capture probes that target an X-linked zinc finger (Zfx) gene and a Y-linked zinc finger (Zfy) gene.

7. The method of claim 1, wherein a female fetal DNA fraction ($f$) of the maternal serum DNA is obtained by using a distribution of an SNP allele frequency formed over an allele frequency range from 0.02 to 0.3, with 0.1 as a median in a non-variation region.

8. The method of claim 1, wherein the monogenic disorder is selected from among Duchenne muscular dystrophy, Pelizaeus-Merzbacher disease, myotubular myopathy, Lowe syndrome, Menkes syndrome, X-linked adrenoleukodystrophy, Hoyeraal-Hreidarsson syndrome, spinal muscular atrophy, metachromatic leukodystrophy, and Krabbe disease.

9. A method for providing diagnostic information for a fetal monogenic disorder, comprising determining a deletion- or duplication-type genetic mutation in a single gene linked to a fetal monogenic disorder by use ofthe maternal serum DNA of any one of claims 1 to 8.

10. A method for detecting a fetal genetic variation, using maternal serum DNA, comprising:

   defining a genetic mutation type of deletion or duplication and a variation region in a single gene linked to a maternal monogenic disorder using the sequencing data of maternal serum DNA;
   obtaining a serum read depth in a variation region and a serum read depth in a non-variation region in the single gene of maternal serum DNA, using sequencing analysis data of maternal serum DNA of a pregnant woman having the genetic variation;
   obtaining a corpuscle read depth in a variation region and a serum read depth in a non-variation region in the single gene of maternal serum DNA, using sequencing analysis data of maternal serum DNA of a pregnant woman having no genetic variation;
   calculating a first ratio of the serum read depth to the corpuscle read depth (serum read depth/corpuscle read depth) in the variation region of the single gene and a second ratio of the serum read depth to the corpuscle read depth (serum read depth/corpuscle read depth) in the non-variation region of the single gene; and
   determining whether or not a fetus has a genetic mutation of deletion or duplication in the single gene linked to a monogenic disorder, by comparing the first ratio with the second ratio.

11. The method of claim 10, comprising
   analyzing maternal serum DNA to give sequencing data;
   defining a genetic mutation type of deletion or duplication and a variation region in a single gene linked to a maternal monogenic disorder using the sequencing data of maternal serum DNA;
   obtaining a serum read depth in a variation region and a serum read depth in a non-variation region in the single gene of maternal serum DNA, using sequencing analysis data of maternal serum DNA of a pregnant woman having the genetic variation;
   analyzing a maternal corpuscle DNA of a pregnant woman having the genetic variation to give sequencing data;
   obtaining a corpuscle read depth in a variation region and a serum read depth in a non-variation region in the single gene of maternal serum DNA, using sequencing analysis data of maternal serum DNA of a pregnant woman having no genetic variation;
   calculating a first ratio of the serum read depth to the corpuscle read depth (serum read depth/corpuscle read depth) in the variation region of the single gene and a second ratio of the serum read depth to the corpuscle read depth (serum read depth/corpuscle read depth) in the non-variation region of the single gene; and
   determining whether or not a fetus has a genetic mutation of deletion or duplication in the single gene linked to a monogenic disorder, by comparing the first ratio with the second ratio.

12. The method of claim 10, wherein the step of analyzing maternal serum DNA is carried out by obtaining the maternal serum DNA and sequencing the serum DNA.

13. The method of claim 10, wherein the step of analyzing maternal corpuscle DNA is carried out by obtaining the maternal corpuscle DNA and sequencing the corpuscle DNA.

14. The method of claim 12 or 13, wherein the maternal serum DNA and corpuscle DNA are obtained by sampling maternal blood, separating the blood into serum and corpuscles, and isolating respective DNAs from the serum and the corpuscles.

15. The method of claim 12 or 13, wherein the maternal serum DNA and the maternal corpuscle DNA are analyzed using a next-generation sequencing method including target enrichment and massively parallel sequencing.

16. The method of claim 12 or 13, wherein the maternal serum DNA and the maternal corpuscle DNA are analyzed by a next-generation sequencing method using identical custom capture probes under identical analysis conditions.

17. The method of claim 10, wherein the step of defining a genetic mutation type of deletion or duplication and a variation region in a single gene is performed by calculating a moving average from a read depth obtained from analysis of maternal serum DNA through next-generation sequencing and by applying CBS (circular binary segmentation).

18. The method of claim 10, wherein the first ratio is a mean or median value of two or more ratio of serum read depth/corpuscle read depth obtained from individual nucleotides in the variation region of the single gene, and the second ratio is a mean or median value of two or more serum read depth/corpuscle read depth obtained from individual nucleotides in the non-variation region of the single gene.

19. The method of claim 10, wherein the first ratio is a mean or median value of two or more serum read depth/corpuscle read depth of the moving average of read depths obtained from individual nucleotide bins of 5 to 100,000 bases in the variation region in the single gene, and the second ratio is a mean or median value of two or more serum read depth/corpuscle read depth of the moving average of read depths obtained from individual nucleotide bins of 5 to 100,000 bases in the non-variation region in the single gene.

20. The method of claim 10, wherein, when an X-linked monogenic disorder is analyzed in a pregnant woman carrying a male fetus, the fetus is determined to have a deletion mutation in the single gene if the maternal variation is of a deletion type and the first ratio in the variation region is smaller than that in the non-variation region.

21. The method of claim 10, wherein, when an X-linked monogenic disorder is analyzed in a pregnant woman carrying a male fetus, the fetus is determined to have a duplication mutation in the single gene if the maternal variation is a type of duplication and the first ratio in the variation region is larger than that in the non-variation region.

22. The method of claim 10, wherein the monogenic disorder is an X-linked monogenic disorder.

23. The method of claim 10, wherein the monogenic disorder is selected from among Duchenne muscular dystrophy, Pelizaeus-Merzbacher disease, myotubular myopathy, Lowe syndrome, Menkes syndrome, X-linked adrenoleukodystrophy, Hoyeraal-Hreidarsson syndrome, spinal muscular atrophy, metachromatic leukodystrophy, and Krabbe disease.

24. A method for providing information for diagnosis of a fetal monogenic disorder, comprising by determining a deletion- or duplication-type genetic mutation in a fetus single gene by use of the maternal serum DNA of any one of claims 10 to 23, to account for the fetal monogenic disorder.

**FIG. 1**

Expected allele frequency in a duplication region

**FIG. 2**

cfDMD01 17wk

## INTERNATIONAL SEARCH REPORT

| International application No. |
| --- |
| **PCT/KR2015/007461** |

| A. | CLASSIFICATION OF SUBJECT MATTER |
| --- | --- |

*G06F 19/22(2011.01)i, C12Q 1/68(2006.01)i*

According to International Patent Classification (IPC) or to both national classification and IPC

| B. | FIELDS SEARCHED |
| --- | --- |

Minimum documentation searched (classification system followed by classification symbols)
G06F 19/22; G06F 19/16; G06F 19/18; G06F 19/10; C12Q 1/68; C40B 30/04

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: mother, fetal, blood serum DNA, single-gene, genetic variation, allele, prediction value, read depth

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-2010-0058503 A (THE CHINESE UNIVERSITY OF HONG KONG) 03 June 2010<br>See abstract; claims 1-23. | 1-23 |
| A | KR 10-2014-0023847 A (BGI HEALTH SERVICE CO., LTD.) 27 February 2014<br>See abstract; claims 30-40. | 1-23 |
| A | WO 2014-033455 A1 (ZORAGEN BIOTECHNOLOGIES LLP.) 06 March 2014<br>See abstract; claims 1-17. | 1-23 |
| A | US 2011-0086769 A1 (OLIPHANT, A. R. et al.) 14 April 2011<br>See abstract; claims 1-24. | 1-23 |
| A | WO 2014-043763 A1 (THE CHINESE UNIVERSITY OF HONG KONG) 27 March 2014<br>See abstract; claims 116-134. | 1-23 |

| ☐ Further documents are listed in the continuation of Box C. | ☒ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 27 OCTOBER 2015 (27.10.2015) | **27 OCTOBER 2015 (27.10.2015)** |

| Name and mailing address of the ISA/**KR** | Authorized officer |
| --- | --- |
| Korean Intellectual Property Office<br>Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (July 2009)

## INTERNATIONAL SEARCH REPORT

| International application No. |
| --- |
| **PCT/KR2015/007461** |

---

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☒ Claims Nos.: **24**
because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

INTERNATIONAL SEARCH REPORT
Information on patent family members

International application No.

**PCT/KR2015/007461**

| Patent document cited in search report | Publication date | Patent family member | Publication date |
|---|---|---|---|
| KR 10-2010-0058503 A | 03/06/2010 | AU 2008-278839 A1 | 29/01/2009 |
| | | AU 2008-278843 A1 | 29/01/2009 |
| | | CA 2693081 A1 | 29/01/2009 |
| | | CA 2694007 A1 | 29/01/2009 |
| | | CN 101849236 A | 29/09/2010 |
| | | CN 101971178 A | 09/02/2011 |
| | | CN 101971178 B | 26/03/2014 |
| | | CN 103849684 A | 11/06/2014 |
| | | CN 103853916 A | 11/06/2014 |
| | | CN 103902809 A | 02/07/2014 |
| | | EP 2183692 A1 | 12/05/2010 |
| | | EP 2183693 A1 | 12/05/2010 |
| | | EP 2183693 B1 | 01/01/2014 |
| | | EP 2514842 A2 | 24/10/2012 |
| | | EP 2514842 A3 | 26/12/2012 |
| | | EP 2527471 A2 | 28/11/2012 |
| | | EP 2527471 A3 | 26/12/2012 |
| | | EP 2557517 A2 | 13/02/2013 |
| | | EP 2557517 A3 | 05/11/2014 |
| | | EP 2557518 A2 | 13/02/2013 |
| | | EP 2557518 A3 | 08/10/2014 |
| | | EP 2557519 A2 | 13/02/2013 |
| | | EP 2557519 A3 | 23/07/2014 |
| | | EP 2557520 A2 | 13/02/2013 |
| | | EP 2557520 A3 | 29/04/2015 |
| | | JP 05519500 B2 | 11/06/2014 |
| | | JP 05736170 B2 | 17/06/2015 |
| | | JP 2010-534068 A | 04/11/2010 |
| | | JP 2010-534069 A | 04/11/2010 |
| | | JP 2014-073134 A | 24/04/2014 |
| | | JP 2015-142588 A | 06/08/2015 |
| | | KR 10-2010-0075826 A | 05/07/2010 |
| | | US 2009-0029377 A1 | 29/01/2009 |
| | | US 2010-0112590 A1 | 06/05/2010 |
| | | WO 2009-013492 A1 | 29/01/2009 |
| | | WO 2009-013496 A1 | 29/01/2009 |
| KR 10-2014-0023847 A | 27/02/2014 | CA 2791118 A1 | 29/12/2012 |
| | | CN 103403183 A | 20/11/2013 |
| | | CN 103403183 B | 15/10/2014 |
| | | EP 2561103 A1 | 27/02/2013 |
| | | EP 2561103 B1 | 27/08/2014 |
| | | JP 05659319 B2 | 28/01/2015 |
| | | JP 2014-520509 A | 25/08/2014 |
| | | US 2014-0099642 A1 | 10/04/2014 |
| | | WO 2013-000100 A1 | 03/01/2013 |
| WO 2014-033455 A1 | 06/03/2014 | CA 2883464 A1 | 06/03/2014 |
| | | EP 2890813 A1 | 08/07/2015 |

Form PCT/ISA/210 (patent family annex) (July 2009)

INTERNATIONAL SEARCH REPORT
Information on patent family members

International application No.

**PCT/KR2015/007461**

| Patent document cited in search report | Publication date | Patent family member | Publication date |
|---|---|---|---|
| | | GB 201215449 D0 | 17/10/2012 |
| | | KR 10-2015-0070111 A | 24/06/2015 |
| US 2011-0086769 A1 | 14/04/2011 | AU 2009-329946 A1 | 11/08/2011 |
| | | CA 2748030 A1 | 01/07/2010 |
| | | CN 102325901 A | 18/01/2012 |
| | | CN 104531837 A | 22/04/2015 |
| | | EP 2379746 A1 | 26/10/2011 |
| | | JP 2012-513217 A | 14/06/2012 |
| | | KR 10-2011-0137286 A | 22/12/2011 |
| | | SG 172345 A1 | 28/07/2011 |
| | | US 9051602 B2 | 09/06/2015 |
| | | WO 2010-075459 A1 | 01/07/2010 |
| WO 2014-043763 A1 | 27/03/2014 | CA 2884066 A1 | 27/03/2014 |
| | | EP 2898100 A1 | 29/07/2015 |
| | | KR 10-2015-0082228 A | 15/07/2015 |
| | | PH 12015500547 A1 | 04/05/2015 |
| | | TW 201418474 A | 16/05/2014 |
| | | US 2014-0080715 A1 | 20/03/2014 |

Form PCT/ISA/210 (patent family annex) (July 2009)

## REFERENCES CITED IN THE DESCRIPTION

**Non-patent literature cited in the description**

- **LO YM et al.** *Science Translational Medicine,* 2010, vol. 2, 61ra91
- **LIAO GJ et al.** *Clinical Chemistry,* 2011, vol. 57, 92-101
- **KITZMAN JO et al.** *Science Translational Medicine,* 2012, vol. 4, 137ra76